# EUROPEAN PATENT APPLICATION

(11) **EP 4 575 057 A1**
(43) Date of publication of application: **25.06.2025**
(21) Application number: 23218688.2
(22) Date of filing: 20.12.2023
(51) Int. Cl.: D04B 1/10, D04B 1/12

(54) **KNITTED ARTICLE, IN PARTICULAR FOR AN ORTHOPAEDIC BRACE**

(71) Applicant: F.G.P. S.R.L., 37062 Villafranca Di Verona (Verona) (IT)
(72) Inventor: FERRIGOLO, Moreno, 37062 Villafranca di Verona (VR) (IT); TURRINI, Alberto, 37062 Villafranca di Verona (VR) (IT); ZAMPINI, Giacomo, 37062 Villafranca di Verona (VR) (IT); REGGIO, Marco, 37062 Villafranca di Verona (VR) (IT)
(74) Representative: Sandri, Sandro

(57) **Abstract**

A knitted article, in particular for an orthopaedic brace, including at least one flat-knitted zone and at least one tunnel zone suitable for the integration of functional elements, such as tensioning cables, comprises a lower layer consisting of several rows of knitted stitches obtained by knitting a first flat-knitted thread, and an upper layer consisting of several rows of knitted stitches obtained by knitting a second flat-knitted thread. The flat-knitted zone consists of lower and upper layer connected to each other. In particular, the first and second flat-knitted threads are the same type of flat-knitted thread. In addition, the tunnel zone consists of lower layer and upper layer detached from each other to define a containment pocket to allow functional elements to pass and be kept in place.

## Description

### Technical field

The present invention relates to the field of weft knitwear for the production of fabrics or articles that may be useful in the production of braces, bandages, socks or other.

In particular, the invention refers to a knitted article, for example for making an orthopaedic brace, configured to receive functional elements such as tensioning cables.

The invention is also directed to a process for making a knitted article, in particular of the type mentioned above.

### Prior art

In the field of the joint braces and orthoses, for example knee orthoses, there are multiple types of braces as a function of the pathology and the rehabilitation path that a patient must follow to recover, if not all, at least in part, the mobility of the compromised joint.

Generally, the joint braces have a tubular body made of fabric suitably conformed to wrap the joint as a function of the rehabilitation that must be performed. The use of a fabric for making the tubular body stems from the need to provide an article with an adequate wearability and that is not uncomfortable for the patient, discouraging the use thereof.

However, the fabric tubular body alone is not sufficient to ensure the adequate fit and compression on the traumatized muscle and joint bands that need to be rehabilitated. Therefore, it is known to apply on the braces one or more functional elements that can confer greater rigidity and/or compression, such as tensioning cables, plates and/or bars made of plastic or metal.

Currently, these functional elements are applied to the textile base of the brace with subsequent application of containment pockets and/or paddings which are useful for cushioning the possible impacts, during use of the brace, of the same functional elements against the patient's soft tissues.

Such pockets and/or paddings are usually applied by means of techniques such as sewing, high-frequency welding, or other welding techniques.

The use of these techniques for making braces is not without drawbacks.

The welding of components on the textile base is usually carried out by means of high-frequency welding or heat-sealing techniques with which it is possible to couple two elements having different mechanical properties since they are made of different materials. This, therefore, involves an alteration of the mechanical behaviour of the entire brace. In addition, even if two components made of the same material are welded together, the glues or the welding films used to define the union of the previously mentioned components alter the mechanical behaviour of the brace in the zones where they are applied.

Sewing external elements is an alternative technique for the application of external components to the base textile structure. In the event that the stitched components are made with a material different from that of the base textile structure, the same drawbacks described above occur, that is, also in this case the same effects of alteration of the mechanical properties of the entire brace occur. In general, the seams tend to alter the mechanical behaviour of the brace even if the same material is used to make the textile base and the components applied thereto. Finally, the seams can undergo wear and, therefore, define points of criticality and breakage that lead to a reduction in the operating life of the brace.

Document EP3480350 describes a knitted article subdivided into zones with different cushioning properties, in particular for making a sole of a footwear. In particular, the previously mentioned knitted article is made as a pair of layers knitted in together, each of which is obtained by the processing of a weft thread. As a function of the cushioning that is to be conferred to a specific zone of the knitted article, the previously mentioned two layers are knitted in with more or less dense meshes in such a way that a further layer interposed between them, made with the same weft thread, is more or less free to define the padding of the knitted article.

However, it is immediately apparent that the knitted article in question is not suitable to act as an orthopaedic brace, or as a similar article, as it has a high rigidity because of the presence of an intermediate layer throughout its extension. Furthermore, this also results in a higher total weight and less breathability.

### Summary

In this context, the technical task underlying the present invention is to propose a knitted article, in particular for an orthopaedic brace, and a process for making a knitted article that overcome the aforementioned drawbacks of the prior art.

In particular, an object of the present invention is to provide a knitted article, in particular for an orthopaedic brace, which is easily wearable by a patient and, moreover, which is comfortable and breathable once worn. In other words, it is intended to provide a knitted article that has an optimal wearability.

Another object of the present invention is to provide a knitted article, in particular for an orthopaedic brace, made by saving material without compromising the operating functionalities thereof. Therefore, specifically, the invention aims to reduce the final weight of the knitted article to increase the wearability by the user and, furthermore, to reduce its production costs.

A further object of the present invention is to provide a knitted article, in particular for an orthopaedic brace, comprising any external components applied without compromising the mechanical properties of the possible orthopaedic brace and moreover, stably positioned on the brace.

Finally, an object of the present invention is to provide a process for making a knitted article, in particular for an orthopaedic brace, which does not provide for the application of external elements through welding and/or sewing techniques such that the final mechanical properties of the knitted article could be compromised.

In addition, the previously mentioned process also aims to control with precision the positioning of any external and functional components to the brace with respect to the base structure itself of the knitted article, without altering the final mechanical behaviour.

The stated technical task and specified objects are substantially achieved by a knitted article, in particular for an orthopaedic brace, and a process for making a knitted article, which comprise the technical features disclosed in the respective independent claims. The dependent claims correspond to further advantageous aspects of the invention.

It should be appreciated that this summary introduces a selection of concepts in simplified form, which will be further elaborated on in the detailed description provided below.

The present invention concerns a knitted article, in particular for an orthopaedic brace, including at least one flat-knitted zone and at least one tunnel zone.

Advantageously, the presence of tunnel zones and of flat-knitted zones allows the previously mentioned knitted article to be used preferably as a bandage, or as a footwear or orthosis for knees or for other joints.

In particular, the previously mentioned knitted article comprises a lower layer consisting of several rows of knitted stitches obtained by knitting a first flat-knitted thread, and an upper layer consisting of several rows of knitted stitches obtained by knitting a second flat-knitted thread.

In greater detail, the flat-knitted zone consists of the lower layer and of the upper layer connected to each other and, preferably, the first and second flat-knitted threads are the same type of flat-knitted thread. The tunnel zone, on the other hand, consists of the lower layer and the upper layer detached from each other to define a containment pocket to allow functional elements to pass and be kept in place.

Advantageously, making a woven fabric (i.e., the knitted article) comprising tunnels integrated into the base structure itself of the woven fabric (i.e., the presence of tunnel zones adjacent to the flat-knitted zone) allows to provide containment pockets wherein functional elements, such as tensioning cables, can be inserted and kept in place without reducing the elastic properties of the knitted article.

In other words, for example in the event that the knitted article is used for making an orthosis, the tunnels are advantageously configured to allow an easy positioning of functional elements to the orthosis itself such as plates made of plastic material, metal rods of the joints, tensioning cables of a winder, straps, etc.

In accordance with an aspect of the invention, the first and the second flat-knitted threads are the same type of flat-knitted thread for the realization of the tunnel zone In accordance with an alternative aspect of the invention, the first flat-knitted thread is different from the second flat-knitted thread for the realization the tunnel zone.

In the event that the first and second flat-knitted threads are of different types, they may have similar mechanical, elastic, and structural properties as the realisation of the tunnel zones must not affect the mechanical properties of the flat-knitted zones, i.e., mechanical properties, elasticity and wearability of the brace made from the above knitted article.

In accordance with one aspect of the invention, the first and second flat-knitted threads comprise natural fibres, including wool, cotton, etc., and/or synthetic fibres including polyester, polyamide, etc.

In accordance with another aspect of the invention, each tunnel zone comprises at least an inlet opening for inserting a functional element into said containment pocket.

Preferably, each tunnel zone comprises an inlet opening and an outlet opening for, respectively, inserting in and extracting - at least partially - from the tunnel a functional element that can able be kept in place inside the same tunnel.

For example, if a tensioning cable - generally made of steel - is to be conveyed, this can be passed through the stitched links of the tunnel itself so that it can be inserted and also subsequently extracted from the opposite end of the tunnel. In fact, the particular small size of a tensioning cables can allow this operation even in the absence of inlet/outlet openings.

However, other functional elements such as plates or others may not allow such an insertion operation.

Therefore, the presence of at least one opening simplifies the positioning and conveying of functional elements within the tunnels.

In accordance with a further aspect of the invention, the lower layer and the upper layer of the tunnel zone are knitted together in several stitching points. In particular, the stitching points defines the contour that delimits the tunnel zone from the adjacent flat-knitted zone.

In other words, the upper and lower layers are totally detached to each other so as not to define any impediment to the placement of functional elements within the corresponding tunnel.

The only points of interconnection are therefore defined by the stitching points where the transition between a flat-knitted zone and a tunnel zone takes place and vice versa, i.e. the points where - in the case of the use of a numerically controlled knitting machine - the change of frontage and/or the change of yarn to be imbedded takes place (in the event that the upper layer of the tunnel zone is made with a different type of flat-knitted thread).

In accordance with one aspect of the invention, the knitted article includes also at least a padded zone having cushioning properties different from the flat-knitted zone and consisting of a padding layer interposed between the lower layer and an intermediate layer. In particular, the padding layer is obtained by a padding yarn which is different from each flat-knitted thread, and which comprises at least one bundle of padding threads. Additionally, the intermediate layer consists of several rows of knitted stitches obtained by knitting the first flat-knitted thread.

Advantageously, making a woven fabric (i.e., the knitted article) comprising paddings integrated into the base structure itself of the woven fabric (i.e., the presence of padded zones adjacent to the flat-knitted zone) allows to provide protection zones for the patient's soft tissues on which the previously mentioned knitted article will be applied. For example, in fact, the knitted article can be configured to make an orthosis for the treatment of knee osteoarthritis wherein the padded zones offer protection to the soft tissues from contact and/or pressure exerted by rigid functional elements of the brace, such as the plates made of plastic material, the metal rods of the joints, the tensioning cable of a winder, straps, etc.

In general, the knitted article in accordance with the invention can serve as a base for different types of orthoses, for example all those orthoses that need paddings adapted to protect the soft tissues of the patients from contact and/or pressure exerted by rigid functional components of the brace in question such as the splints of a corset, the rods of a wristband, or the joints of an elbow pad.

In accordance with one aspect of the invention, each padding yarn comprises at least three bundles of padding threads.

Preferably, each bundle of padding threads can be made by the union of padding threads of different types, for example different synthetic and/or natural fibres, so as to have different elasticity and/or swelling characteristics as a function of the needs of the patient.

Even more preferably, each bundle of padding threads comprises at least three padding threads so as to combine with each other the different mechanical properties of the various types of padding threads used.

In accordance with another aspect of the invention, each thread of the bundle of padding threads is a multi-strand thread.

Preferably, each thread of the bundle of padding threads can be made by the union of different strands so as to confer to the same thread different mechanical properties (for example, in terms of elasticity or swelling) with respect to the others.

Even more preferably, each thread of a bundle of padding threads comprises at least eighteen strands.

Advantageously, therefore, the possibility of selecting threads and/or strands - both with respect to making the flat-knitted zone and the padded zone - allows to customize and adapt the final mechanical properties that the knitted article must possess in order to be optimal for the patient who will have to wear it.

In particular, the selection of the number and type of padding strands and/or of the padding threads for making the yarn of the padding layer allows making padded zones with variable shapes, thicknesses and consistency depending on the type of final padding strand used.

In accordance with an aspect of the invention, the padding yarn comprises natural fibres, including wool, cotton, etc., and/or synthetic fibres including polyester, polyamide, etc.

In accordance with a further aspect of the invention, the padding yarn extends over a number of rows less than the number of rows of knitted stitches of the lower and intermediate layers between which it is arranged.

In other words, as already mentioned, the padded layer is present only at the padded zones and not over the entire succession of rows of knitted stitches with which the lower and upper layers were made.

In further other words, the use of the padding yarn is strictly localized in the padded zones, while in the flat-knitted zones no padding yarn is present between the lower and upper layers.

In this way, the knitted article in accordance with the invention is advantageously able to guarantee a better wearability and breathability to the patient who will use it, thanks to a reduced weight and a greater flexibility. The knitted articles known to the state of the art (with which orthoses are also made) have a padding yarn also in the flat-knitted zones, increasing the rigidity and the overall weight of the knitted article itself, as well as its final cost. In fact, this excessive provision of the padding yarn implies further processing aimed at firmly constraining and squeezing the previously mentioned yarn between the external layers of the brace in order to create a flat-knitted layer which, however, will have greater thickness and rigidity than that obtained with the knitted article in accordance with the invention.

In accordance with one aspect of the invention, a plurality of padding yarns is laid in at least one row of knitted stitches of the lower layer and/or of the upper layer for making the padding layer.

In accordance with a preferred aspect of the invention, the padded zone has a minimum height of 4 millimetres.

In this way, it is advantageously possible to modulate the impact cushioning properties of the knitted article as a function of the needs of the patient, thanks to the variation of the number of padding yarns laid in at least one row of knitted stitches of the lower and/or upper layers.

In accordance with another aspect of the invention, the padding layer of a first padded zone is constrained to the intermediate and lower layers only at at least a part of a contour perimeter delimiting the same padded zone from another padded zone and/or from one or more adjacent flat-knitted zones.

In other words, as a function of the final destination the knitted article will have - that is, whether it will be used as a bandage, or as an orthosis for a specific joint - each padded zone has a specific conformation and, therefore, a specific contour perimeter of predetermined shape. For example, a padded zone may have a circular, square, or irregular conformation.

Consequently, the padded layer is constrained to the lower and/or intermediate layers of a padded zone only at the contour perimeter and, therefore, at the knitting-in stitches that are made between the previously mentioned lower and intermediate layers.

In this way, the padded zone substantially consists of a single chamber with unconstrained external layers (the lower and upper layers) and a padding yarn laid in the aforesaid two layers.

In other words, in each padded zone, the only stitches where the padding yarn is constrained to the flat-knitted threads of the lower and intermediate layers are the knitting-in stitches present along the contour perimeter of the padded zone itself.

Preferably, a padded layer is realised with a padding yarn arranged along a plurality of stitching rows (basically describing a serpentine), i.e., the number of rows defining the padded area. Consequently, for each stitching rows, the ends of the padding yarn are secured to stitches of the lower and/or upper layer as these stitches coincide with the aforementioned contour points of the padded area.

In the event that the knitted article is made with a numerically controlled knitting machine, these knitting-in stitches correspond to the passage of the flat-knitted threads from one needle bed to the other of the textile machine, i.e., the so-called interlacing stitches.

Advantageously, the previously mentioned interlacing stitches can also be applied within the contour perimeter of a padded zone in order to vary the conformation of the padded zone itself, for example to reduce the thickness, the swelling capacity thereof and to increase the overall rigidity thereof. In other words, each padded zone can be subdivided into several mutually adjacent padded zones. These intermediate interlacing stitches, therefore, define further constraint stitches as they trace part of new contour perimeters.

Advantageously, the arrangement of these further interlacing stitches can give rise to two-dimensional figures which, depending on the distance interposed between the various stitches, give rise to a region with padding with differentiated thickness, since there is a positive correlation between distance between the interlacing stitches and the swelling capacity of the padding yarn.

Preferably, the knitted article comprises multiple padded zones arranged in a circumference direction.

In accordance with an aspect of the invention, the padding yarn is laid in at least one row of knitted stitches of the lower layer and/or of the intermediate layer. In particular, the padding yarn is laid into at least one knitted stitch of the lower layer and/or of the intermediate layer.

Preferably, the padded zone is located at a tunnel zone so that each tunnel is knitted onto that padded zone.

More preferably, the tunnel zone extends over a number of rows of knitted stitches less than the number of rows of knitted stitches of the intermediate or lower layer of the padded zone on which it is knitted onto.

In other words, each tunnel is preferably made only at one or more padded zones as the latter must dampen the contact and the pressure generated by the functional elements of the orthosis with the soft tissues of the patient.

Advantageously, each padded zone associated with a tunnel allows to protect the soft tissues of the patient from contact and/or pressure exerted by the previously mentioned rigid functional elements of the orthosis contained and/or guided by the tunnel.

The invention also refers to a kit, preferably for an orthopaedic brace, comprising a knitted article having one or more of the foregoing described features in any combination. Additionally, the kit comprises at least a functional element, such as tensioning cable, suitable to be passed and be kept in place in each tunnel zone of said knitted article, and a guide element configured to be connected to the functional element and to be conveyed through the tunnel zones so as to pass the functional element through the corresponding tunnel.

Preferably, the guide element comprises a first end bevelled to be inserted through each tunnel zone without damaging each flat-knitted threads, and a second end, opposite to the first end, comprising a coupling portion to which the functional element can be connected.

More preferably, the guide element comprises a main body with a cylindrical shape. The second end could have a hole suitable to fix a corresponding piece of a functional element, such as an end of a tensioning cable, in order to simply the conveying of the functional element through each tunnel zone.

The invention also refers to a process for making a knitted article, in particular for an orthopaedic brace, including at least one flat-knitted zone and at least one tunnel zone.

The previously mentioned process comprises the following steps:
- knitting a first flat-knitted thread over several rows of knitted stitches so as to make a lower layer;
- knitting a second flat-knitted thread over several rows of knitted stitches so as to make an upper layer, wherein the flat-knitted thread of the upper layer is alternatively processed jointly with or independently of said lower layer to, respectively, make a flat-knitted zone or a tunnel zone.

In particular, the first and second flat-knitted threads are the same type of flat-knitted thread for realizing each flat-knitted zone.

In other words, the previously mentioned process makes it possible to make a woven fabric (i.e., the knitted article) in which the flat-knitted zones and the tunnel zones are advantageously made with a single production step.

In other words, the tunnel zones are advantageously integrated during the same realization of the flat-knitted zones and, therefore, are not applied later as a patch by welding or sewing as in the prior art.

Even more advantageously, the fact that the tunnel zone is made starting from the same structure as a flat-knitted zone, and preferably with also the same type of flat-knitted thread, allows several functional advantages to be obtained when the knitted article is put into use:
- the mechanical behaviour of the tunnels in terms of elasticity and deformation is perfectly consistent with the rest of the textile structure of the knitted article, especially in the event that the latter is configured as an orthosis;
- the tunnel zones of the knitted article have a high degree of adaptation to the anatomical shapes of the using patients in a manner consistent with the rest of the textile structure, i.e., the flat-knitted zones, since the entire padding layer is made with a yarn having mechanical characteristics consistent with the flat-knitted threads;
- the constitutive nature of the tunnel zones does not alter the behaviour of the flat-knitted zones as the zones do not present discontinuities between them, being woven together with the structure itself.

The foregoing process also comprises a step of realizing a padded zone having cushioning properties different from at least each flat-knitted zone. The padded zone is located at a tunnel zone so that each tunnel is knitted onto that padded zone.

Preferably, the step of realizing a padded zone comprises the step of making a padded layer by laying in at least one row of knitted stitches of the lower layer and/or of an intermediate layer a padding yarn different from said flat-knitted thread. The padding yarn comprising at least one bundle of padding threads, and the intermediate layer is interposed lower and upper layers.

Preferably, the process provides that each thread of the bundle of padding threads is selected from threads of the multi-strand type.

In accordance with one aspect of the invention, a plurality of padding yarns is laid in at least one row of knitted stitches of the lower layer and/or of the upper layer.

In accordance with another aspect of the invention, the padding layer of a first padded zone is constrained to the intermediate and lower layers only at at least a part of a contour perimeter delimiting the same padded zone from another padded zone and/or from one or more flat-knitted zones.

In accordance with a further aspect of the invention, the process also comprises a step of knitting a further thread over several rows of knitted stitches so as to make at least one tunnel. In particular, said tunnel is knitted onto at least one flat-knitted zone and/or onto at least one padded zone so as to define a containment pocket to allow functional elements to pass and be kept in place.

In this way, the previously mentioned process allows to make, in a single step of production of the flat-knitted zone, also one or more hollow tunnels with integrated protection paddings. In other words, at least at the upper layers of the padded zones, corresponding tunnels configured for the insertion and positioning of functional elements, for example, for an orthosis, are made and knitted in. Conversely, at least in the sector of the orthoses, the current prior art envisages using a flat-knitted textile base with subsequent application of protective paddings and of any tunnels by means of techniques such as sewing, high-frequency welding, other generic welding techniques. However, as illustrated above, such techniques are not exempt from introducing negative effects on the elastic behaviour of the textile base.

In accordance with an aspect of the invention, each tunnel is made with an extension of rows of knitted stitches less than the number of rows of knitted stitches of the intermediate layer of the padded or flat-knitted zone on which it is knitted onto.

In accordance with a preferred aspect of the invention, the process also comprises a preliminary step of setting up a numerically controlled textile machine equipped with two distinct needle beds and configured to carry out the aforementioned processing steps so as to produce a woven piece, i.e. a substantially planar knitted article, in which the tunnel zones are integrated on a knitted article, more precisely, integrated with the flat-knitted zones of said knitted article.

In this way, the process is advantageously able to allow making flat-knitted zones and/or padded zones and/or tunnels without requiring any particular modification of the production plant. The numerically controlled knitting machine, in fact, can be correctly configured to vary the activation of the needle beds that compose it and, above all, of the number of needles of the aforesaid needle beds in order to make, in succession to the flat-knitted zones, the padded zones and/or the tunnels.

In addition, the previously mentioned process is also advantageously capable of making padded zones defined by a single chamber for containing the padding layer, without weaving courses completely occupied by the padding yarn and, in addition, without weaving courses completely interposed with two padded zones.

### Brief description of the drawings

Further features and advantages of the present invention will become more apparent from the indicative, and therefore non-limiting, description of a preferred but non-exclusive embodiment of a knitted article, in particular for an orthopaedic brace, as illustrated in the accompanying drawings in which:
- figure 1 illustrates, according to a schematic view, a knitted article in accordance with the present invention;
- figure 2 illustrates, according to a side view, a section of the knitted article of figure 1 along segment A-A;
- figure 3 illustrates, according to a section side view, a further aspect of the knitted article;
- figure 4 illustrates, according to a perspective view, a possible conformation of a tunnel knitted onto a padded zone and a guide element part of a kit according to the present invention;
- figures 5a-5c illustrate, according to different lateral views, several aspects of a brace realized with the knitted article of figure 1.

With reference to the drawings, they serve solely to illustrate embodiments of the invention with the aim of better clarifying, in combination with the description, the inventive principles on which the invention is based.

### Detailed description of at least one embodiment

The present invention is directed to a knitted article, in particular for an orthopaedic brace, which, with reference to the figures, has been generically indicated with the number 1.

Any modifications or variants which, in the light of the description, are evident to the person skilled in the art must be considered to fall within the scope of protection established by the present invention, according to considerations of technical equivalence.

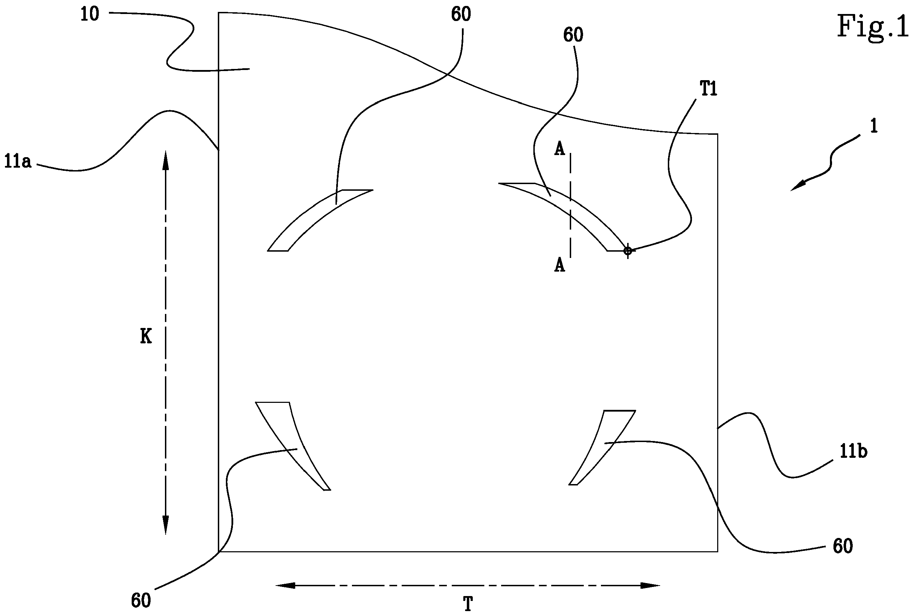

Figure 1 schematically illustrates a knitted article 1, in particular for an orthopaedic brace, with a plurality of zones having different cushioning properties, including at least one flat-knitted zone 10 and at least one tunnel zone 60.

In particular, figure 1 shows that the knitted article 1 has a preferably planar conformation.

Even more preferably, said knitted article 1 is made with a numerically controlled textile machine of the straight type, i.e., capable of making planar woven elements that are open along a working direction K illustrated with a specific exemplary arrow. Figure 1 also illustrates the direction of travel of the carriage and of the thread guides of the previously mentioned textile machine. This direction is indicated by the letter T and is substantially perpendicular to the aforesaid working direction K. In addition, the aforesaid textile machine comprises at least two needle beds facing each other so as to make different types of knitted stitches.

Advantageously, therefore, the knitted article 1 thus obtained can maintain its substantially planar conformation to be used as a bandage, or it can be configured in a three-dimensional structure in order to make, for example, a sock, a footwear, or an orthosis for a joint such as a knee brace.

Even more advantageously, the use of the numerically controlled textile machine makes it possible to adjust the knitting-in and the arrangement of a first and second flat-knitted threads to create contiguous differentiated zones in which zones with flat-knitted 10, zones with tunnel 60 and zones with padding 30 on which a tunnel has been knitted onto (the latter aspect will be better described later) can be alternated. The general shape of the knitted article 1 can therefore be generic, as can be generic the planar geometry of the padded zone 20 and the geometry of the tunnels 60, which can also be generic in shape with respect to the underlying padded zone 20.

In general, in the event that the numerically controlled textile machine is used, it is useful to say beforehand that the number of needles of each needle bed involved in the described processing and also the final number of weaving courses made is strictly dependent on the final conformation that the knitted article 1 must obtain and each flat-knitted zone 10 and/or padded zone 20 and/or tunnel 60.

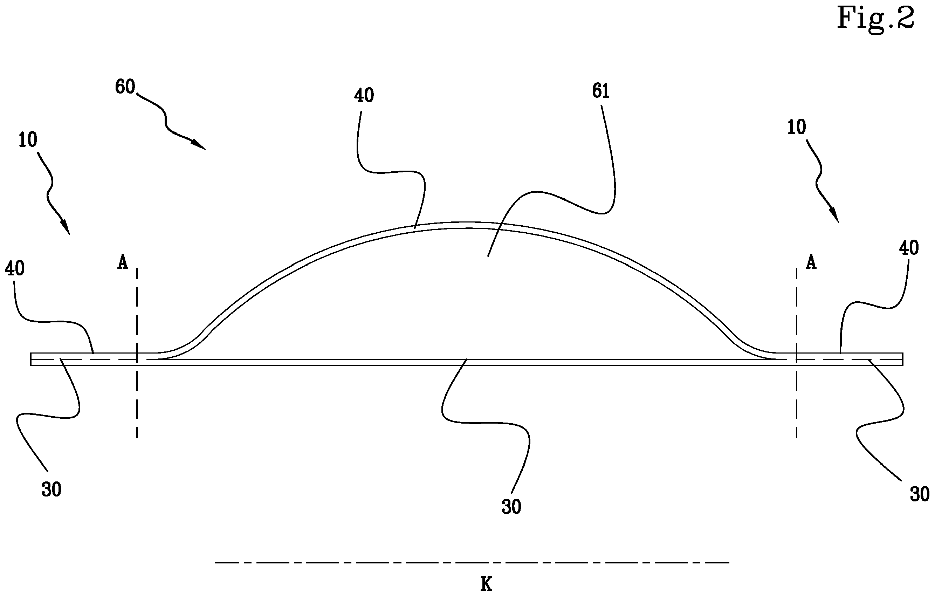

Figure 2 illustrates, according to a side view, further detailed aspects of the previously mentioned knitted article 1.

In particular, the knitted article 1 comprises a lower layer 30 consisting of several rows of knitted stitches obtained by knitting a first flat-knitted thread, and an upper layer 40 consisting of several rows of knitted stitches obtained by knitting a second flat-knitted thread.

The flat-knitted zone 10 consists of the lower layer 30 and of the upper layer 40 connected to each other and, preferably, the first and second flat-knitted threads are the same kind of flat-knitted thread, while the tunnel zone 60 consists of the lower layer 30 and the upper layer 40 detached from each other to define a containment pocket 61 to allow functional elements to pass and be kept in place.

The invention is also directed to a process for making a knitted article 1, in particular for an orthopaedic brace, including at least one flat-knitted zone 10 and at least one tunnel zone 60.

In detail, the previously mentioned process comprises the following steps:
- knitting a first flat-knitted thread over several rows of knitted stitches so as to make a lower layer 30;
- knitting a second flat-knitted thread over several rows of knitted stitches so as to make an upper layer 40. In particular, the flat-knitted thread of the upper layer 40 is alternatively processed jointly with or independently of the lower layer 30 to, respectively, make a flat-knitted zone 10 or a tunnel zone 60 so as to define a containment pocket to allow functional elements to pass and be kept in place.

In particular, the first and second flat-knitted threads are the same type of flat-knitted thread for realizing each flat-knitted zone 10.

Differently, for the realisation of each tunnel zone, the first and second flat-knitted threads may belong to the same type or to two similar but not identical types of flat-knitted threads.

If the first and second flat-knitted threads are different, it is preferable that the mechanical and elastic properties are not excessively different but are comparable so that the construction of the tunnels 60 does not reduce the mechanical and elastic properties of the flat-knitted zones 10 and, therefore, the functionality and fit of the brace made from the foregoing knitted article 1.

Advantageously, the above-mentioned process makes it possible to produce a knitted article 1 in which the tunnels 60 are made in a single operation step directly from the flat-knitted zones 10. In other words, the aforementioned process makes it possible not to have to apply tunnel zones 60 to the flat-knitted zones 10 by stitching and/or by other similar techniques which, as mentioned above, have drawbacks.

Figure 2 illustrates a first cross-section of the knitted article 1 illustrated in figure 1, in particular along segment A-A.

In detail, from left to right, figure 2 illustrates two flat-knitted zones 10 between which a tunnel zone 60 is interposed.

The flat-knitted zones 10 are obtained by processing the first flat-knitted thread for the lower layer 30 and the second flat-knitted thread for the upper layer 40 alternately from the front needle bed to the rear needle bed of a textile machine. In particular, as already exposed, the first and the second flat-knitted thread are the same type of flat-knitted thread. Consequently, in the flat-knitted zones 10 the lower layer 30 and the upper layer 40 are joined together.

At the tunnel zone 60, instead, there are two contiguous layers that are not mechanically constrained:
- a lower layer 30 knitted in from the rear/front needle bed by processing the first flat-knitted thread;
- the upper layer 40 knitted in from the rear/front needle bed by processing the second flat-knitted thread which could be the same or different with respect of said first flat-knitted thread.

In accordance with another aspect of the invention, the process comprising also a step of realizing a padded zone having cushioning properties different from at least each flat-knitted zone. In particular, each padded zone is located at a tunnel zone so that each tunnel is knitted onto that padded zone.

In more detail, the step of realizing a padded zone comprises the step of making a padded layer by laying in at least one row of knitted stitches of the lower layer and/or of an intermediate layer a padding yarn different from said flat-knitted thread. The padding yarn comprising at least one bundle of padding threads, and, in addition, the intermediate layer is interposed between lower and upper layers.

In particular, the process does not provide for arranging any padding yarn between the lower and upper layers 30, 40 of any flat-knitted zones 10.

In other words, for example in the event that a numerically controlled textile machine is used, the padding yarn is deposited between the lower and upper layers 30, 40 by sliding the dedicated thread guide carriage only for the fraction of the course of interest that is comprised between the interlacing stitches that delimit the corresponding padded zone 20.

A preferred aspect of the invention, both with reference to the knitted article 1 and to the process for making a knitted article 1, envisages using substantially two types of thread for making the flat-knitted zones 10 and the padded zones 20 (the tunnels 60 are preferably made with a flat-knitted thread, but they can be made with a similar thread).

Preferably, the first and second flat-knitted thread is a thread selectable among the threads made of a material of natural origin, such as cotton, wool, etc., or among the threads made of a synthetic material, such as polyester, polyamide, etc.

Conversely, the padding yarn has instead a more complex conformation than the flat-knitted thread. The padding yarn, in fact, must have different properties and, in particular, it must ensure that it has a good swelling capacity, e.g., following a washing and drying process. The padded zone, compared to the flat-knitted zone, must have a greater thickness and a much softer consistency as the padded zone has the main function of protecting the patient's soft tissues on which the knitted article is applied. Preferably, then, the padding yarn comprises a bundle of padding threads - preferably three or more distinct bundles twisted together.

Even more preferably, each padding thread of each bundle of padding threads is of the multi-strand type.

In this way, the padding layer is highly customizable as a function of the needs of the end user thanks to the possibility of selecting different strands - in terms of number and/or type - for the formation of each padding thread and, in addition, thanks to the possibility of selecting different padding threads - again in terms of number and/or type - for the formation of the bundles of padding threads that make up the padding strand at the base of the padding layer.

In fact, the use of a bundle of multi-strand threads for making the padding yarn has an effect improving the growth of the yarn itself during the swelling phase.

Even more advantageously, in the event that a numerically controlled textile machine is used for making the knitted article 1, it is possible to adjust the knitting-in and the arrangement of the flat-knitted thread and of the padding yarn in order to create contiguous differentiated zones in which zones with or without padding yarn can be alternated, i.e. the padded zone 30 and the flat-knitted zone 20.

As anticipated, on the other hand, the further thread used to make a tunnel 60 has mechanical characteristics (such as composition, count, elasticity) similar to the flat-knitted thread used to make the flat-knitted zones 10.

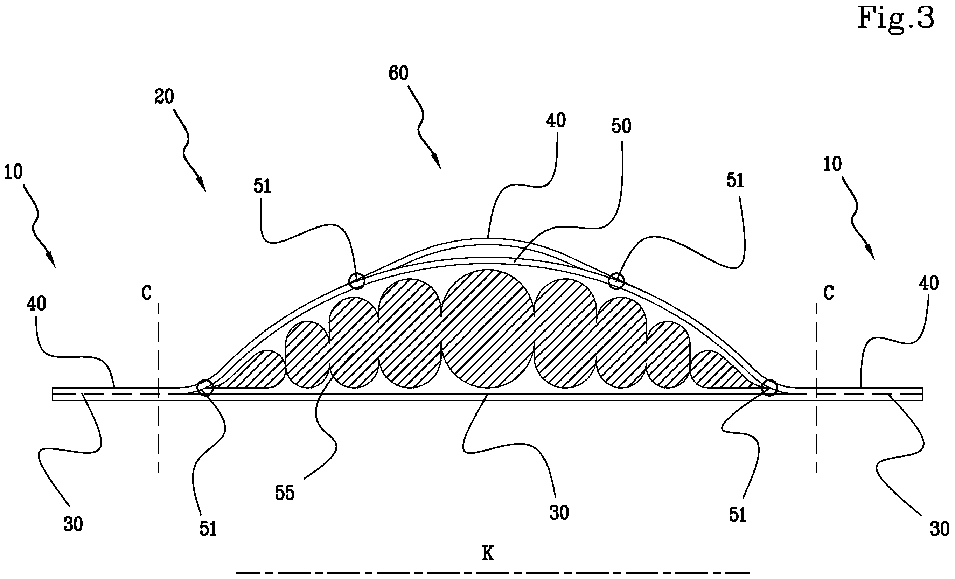

Figure 3 illustrates a further cross-section of an aspect of the knitted article 1 not illustrated in figure 1.

In detail, figure 3 illustrates a padded zone 20 interposed between two flat-knitted zones 10 and on which a tunnel zone 60 is knitted onto.

The flat-knitted zones 10 are made by processing the first flat-knitted thread, preferably by means of a numerically controlled textile machine configured to continuously pass from the front needle bed to the rear needle bed of the machine in an alternating manner.

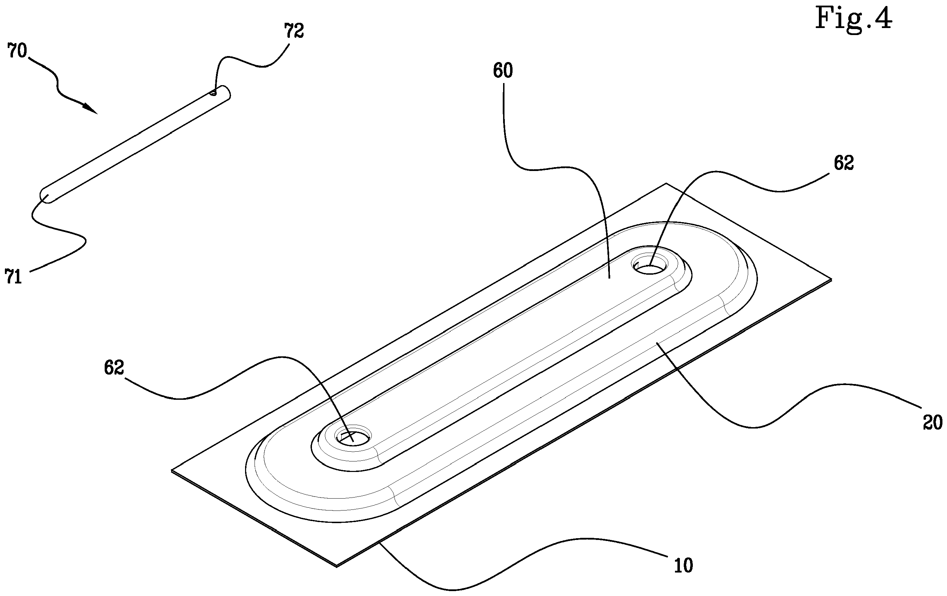

In figure 4 the tunnel zone 60 is knitted onto a padded zone 20 and, therefore, four different contiguous layers can be recognized that are not mechanically constrained over their entire length:
- the lower layer 30 made with the first flat-knitted thread preferably knitted in from the rear needle bed of a numerically controlled textile machine;
- the intermediate layer 50 made with the second flat-knitted thread (equal to the first flat-knitted thread) preferably knitted in from the front needle bed of a numerically controlled textile machine;
- the padded layer 55 consisting of a plurality of padding yarns arranged parallel and lying (not knitted in) between the aforesaid lower and intermediate layers 30, 50;
- the tunnel 60 preferably made with the second flat-knitted thread (not necessarily equal to the first flat-knitted thread) preferably knitted in from the same front needle bed of the numerically controlled textile machine but unconstrained from the upper layer 40.

In detail, the aforesaid layers are knitted in together only at some specific connection stitches, i.e. at the interlacing stitch 51 between the flat-knitted zone 10 and the padded zone 20, and vice versa, to constrain the padding yarn to the lower and upper layers 30, 40 and, in addition, at the two knitting-in stitches of the tunnel 60 with the intermediate layer 40 of the padded zone 20. These two knitting-in stitches also define the stitches that delimit the tunnel.

Preferably, as visible in figure 3, the tunnel 60 has an extension lower than the extension of the padded zone 20, since the padded layer has a main function of protecting the patient's skin with respect to any functional element housed in the tunnel 60 itself. Therefore, in order to be able to effectively perform the previously mentioned function, the padded layer 55 must preferably exceed the extension of the tunnel 60.

Figure 4 illustrates a preferred embodiment of a tunnel 60 knitted in on a padded zone 20 of a knitted article 1 and, additionally, a guide element 70 useful to convey a functional element, such as a tensioning wire, through a tunnel 60.

Preferably, the guide element 70 has a hemispherical end 71, the radius of which has a specific value suitable, on the one hand, to allow the entry and sliding of the guide element 70 into the tunnel 60, and on the other hand suitable not to allow the object to insert itself into the fabric meshes of the intermediate layer 50 interposed between the tunnel 60 and the possible padded layer 55. The guide element 70 has a length comparable to that of the tunnels 60 in which it is to slide, and the central section of the structure is cylindrical with a radius of the section coinciding with that of the semi-sphere 71. The end 72 opposite the hemisphere presents a coupling site for the wire and/or other functional elements to be passed through or inserted into the tunnel 60. Specifically, this site consists of a cylindrical cavity within the volume of the tool, with a wall of suitable thickness and predetermined length. In addition, there is a hole with a transverse direction to the development of the tool, to allow entry of the wire from outside the volume into said cavity. This binds the wire to the tool. The passage of the guide element 70 through the tunnel 60 along its entire length results in the wire itself passing through the tunnel 60.

According to another aspect illustrated in figure 4, the tunnel 60 illustrated in figure 4 also provides for making at least one opening 62, preferably an inlet and an outlet opening 62.

The opening 62, in fact, is advantageously able to facilitate the entry and the exit of the functional elements with respect to the tunnel 60.

Advantageously, the tunnel 60 having the characteristics described above and obtained with the claimed process has a mechanical behaviour, in terms of elasticity and deformation, absolutely consistent with the rest of the structure of the knitted article 1. In this way, the tunnel 60 is able to adapt to the anatomical shapes of the patients on which the knitted article 1 is applied in a manner consistent with the rest of the textile structure. The entire totality of the knitted article 1, in fact, is made of threads and yarns with mechanical characteristics comparable to each other. In addition, even more advantageously, the direct knitting-in of the tunnel 60 on the flat-knitted and/or padded zones 10, 20 does not alter the mechanical behaviour of the entire knitted article 1 as might do any elements fixed at a later time by sewing or welding, such as patches in accordance with the state of the art.

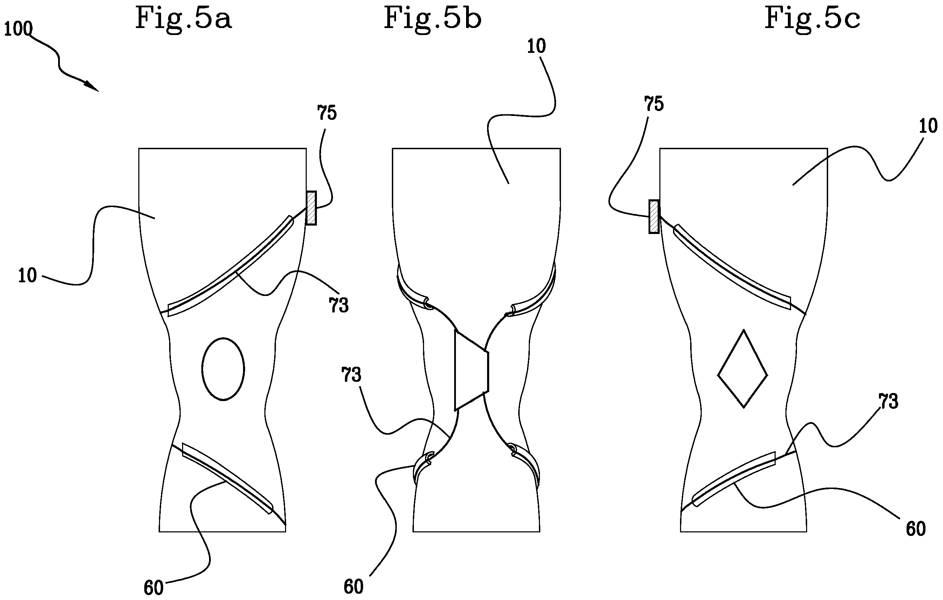

Figures 5a, 5b and 5c illustrate, according to different lateral views, several aspects of a brace 100 realized with the knitted article 1 describe above.

In particular, the brace 100 is a knee brace comprising a main flat-knitted zone 10 on which a plurality, preferably four, tunnels zone 60 are made. A spool 75 connected to a steel tensioning cable 73 is affixed to the brace 100, which has been previously conveyed through the aforementioned tunnels 60 by means of the guide element 70 (illustrated in Figure 4).

Particularly, the spool 75 is a winding system positioned and fixed to the main knitted base of the brace 100 by means of a special interface, capable of tensioning a tensioning wire 73 that follows a specific path developed on the foregoing main knitted base of the brace 100 itself. The applied tensioning wire 73 is unique and, thanks to the winding system, allows the brace 100 to perform its function by adjusting its tension.

The fundamental characteristic of the tunnel 60 is its elasticity. The mechanical behaviour of the tunnel 60 in terms of elasticity and deformation is perfectly consistent with the rest of the main knitted base of the brace 100. In this way, the tunnel 60 is able to adapt to the anatomical shapes of the user patients in a manner consistent with the rest of the main knitted base, because the tunnel 60 is made of threads with mechanical characteristics consistent with the rest of the brace 100. In addition, the constitutive nature of the tunnel 60 does not alter the behaviour of the rest of the main knitted base of the brace 100, as it is knitted together with the structure itself. The tensioning wire 73 inserted inside the tunnels 60 is not mechanically constrained to them, so that the tunnels 60 can adapt to the patient's shape without affecting the cable's movement.

## Claims

1. A knitted article, in particular for an orthopaedic brace, including at least one flat-knitted zone and at least one tunnel zone suitable for the integration of functional elements, such as tensioning cables, comprising:
- a lower layer consisting of several rows of knitted stitches obtained by knitting a first flat-knitted thread;
- an upper layer consisting of several rows of knitted stitches obtained by knitting a second flat-knitted thread;
wherein said flat-knitted zone consists of said lower layer and said upper layer connected to each other, said first and second flat-knitted threads being the same type of flat-knitted thread, and
wherein said tunnel zone consists of said lower layer and said upper layer detached from each other to define a containment pocket to allow functional elements to pass and be kept in place.

2. The knitted article according to claim 1, wherein said first and second flat-knitted threads are the same kind of flat-knitted thread at said tunnel zone.

3. The knitted article according to claim 1, wherein said first flat-knitted thread is different from said second flat-knitted thread at said tunnel zone.

4. The knitted article according to any preceding claim, wherein each tunnel zone comprises at least an inlet opening for inserting a functional element into said containment pocket.

5. The knitted article according to any preceding claim, wherein said first and second flat-knitted threads comprise natural fibres, including cotton and wool, and/or synthetic fibres including polyester and polyamide.

6. The knitted article according to any preceding claim, wherein said lower layer and said upper layer of the tunnel zone are knitted together in several stitching points, said stitching points defining the contour that delimits the tunnel zone from the adjacent flat-knitted zone.

7. The knitted article according to any preceding claim, including at least a padded zone having cushioning properties different from said flat-knitted zone and consisting of a padding layer interposed between said lower layer and an intermediate layer,
said padding layer being obtained by a padding yarn which is different from each flat-knitted thread, and which comprises at least one bundle of padding threads,
said intermediate layer consisting of several rows of knitted stitches obtained by knitting said first flat-knitted thread.

8. The knitted article according to claim 7, wherein said padding yarn is laid in at least one row of knitted stitches of the lower layer and/or of the upper layer, said padding yarn being laid into at least one knitted stitch of the lower layer and/or of the upper layer.

9. The knitted article according to claim 7 or 8, wherein said padded zone is located at a tunnel zone so that each tunnel is knitted onto that padded zone.

10. The knitted article according to claim 9, wherein said tunnel zone extends over a number of rows of knitted stitches less than the number of rows of knitted stitches of the intermediate or lower layer of the padded zone on which it is knitted onto.

11. Kit for an orthopaedic brace, comprising:
- a knitted article according to any preceding claims from1 to 10;
- at least a functional element, such as tensioning cable, suitable to be passed and be kept in place in each tunnel zone of said knitted article;
- a guide element configured to be connected to the functional element and to be conveyed through the tunnel areas so as to pass the functional element through the same tunnel areas.

12. Kit according to claim 11, wherein said guide element comprises
a first end bevelled to be inserted through each tunnel zone without damaging each flat-knitted threads, and
a second end, opposite to the first end, comprising a coupling portion to which the functional element can be connected.

13. A process for making a knitted article, in particular for an orthopaedic brace, including at least one flat-knitted zone and at least one tunnel zone, comprising the following steps:
- knitting a first flat-knitted thread over several rows of knitted stitches so as to make a lower layer;
- knitting a second flat-knitted thread over several rows of knitted stitches so as to make an upper layer, wherein said flat-knitted thread of the upper layer is alternatively processed jointly with or independently of said lower layer to, respectively, make a flat-knitted zone or a tunnel zone so as to define a containment pocket to allow functional elements to pass and be kept in place;
wherein said first and second flat-knitted threads being the same type of flat-knitted thread at each flat-knitted zone.

14. The process according to claim 13, comprising a step of realizing a padded zone having cushioning properties different from at least each flat-knitted zone, said padded zone being located at a tunnel zone so that each tunnel is knitted onto that padded zone, said step of realizing a padded zone comprising the step of making a padded layer by laying in at least one row of knitted stitches of the lower layer and/or of an intermediate layer a padding yarn different from said flat-knitted thread, said padding yarn comprising at least one bundle of padding threads, said intermediate layer being interposed lower and upper layers.

15. The process according to claim 14, wherein each tunnel is made with an extension of rows of knitted stitches less than the number of rows of knitted stitches of the intermediate layer of the padded or flat-knitted zone on which it is knitted onto.
